Europäisches Patentamt

European Patent Office

Office européen des brevets

(19)

(11) Veröffentlichungsnummer: **0 288 679 B1**

# EUROPÄISCHE PATENTSCHRIFT

(12)

(45) Veröffentlichungstag der Patentschrift: **04.12.91**

(51) Int. Cl.⁵: **A61M 1/00**, //B65D51/16

(21) Anmeldenummer: **88102660.3**

(22) Anmeldetag: **24.02.88**

(54) **Stopfen aus gummielastischem Material für eine Saugdrainageflasche.**

(30) Priorität: 29.04.87 CH 1637/87

(43) Veröffentlichungstag der Anmeldung:
**02.11.88 Patentblatt 88/44**

(45) Bekanntmachung des Hinweises auf die
Patenterteilung:
**04.12.91 Patentblatt 91/49**

(84) Benannte Vertragsstaaten:
**AT DE FR GB IT**

(56) Entgegenhaltungen:
**CH-A- 605 313**
**DE-A- 2 642 213**

(73) Patentinhaber: **Genossenschaft VEBO Solot-**
**hurnische Eingliederungsstätte für Behin-**
**derte**
**Werkhofstrasse**
**CH-4702 Oensingen(CH)**

(72) Erfinder: **Hauri, Hermann**
**Fliederweg 6**
**CH-5600 Lenzburg(CH)**

(74) Vertreter: **Fillinger, Peter, Dr.**
**Rütistrasse 1a**
**CH-5400 Baden(CH)**

## Beschreibung

Die vorliegende Erfindung bezieht sich auf einen Stopfen gemäss dem Oberbegriff des Anspruchs 1.

Ein solcher Stopfen ist beispielsweise in der DE-A 26 42 213 beschrieben. Beim dort beschriebenen Stopfen hat sich in der Praxis erwiesen, dass nach dem Evakuieren der Saugdrainageflaschen durch Erhitzen 5 bis 10 % der Flaschen kein hinreichend hohes oder kein Vakuum aufweisen. Die Ursache für diesen Mangel ist nicht bekannt. Zwei Gründe sind denkbar. Nach dem einen schliesst der Stopfen so dicht, dass er während des Erhitzens die heisse Luft nicht abströmen lässt. Nach dem anderen kann die erhitzte Luft abströmen, doch schliesst beim nachfolgenden Abkühlen die Flasche nicht hinreichend vakuumdicht.

Die vorliegende Erfindung stellt sich die Aufgabe, einen Stopfen der erwähnten Art derart zu verbessern, dass nach dem Evakuieren der Saugdrainageflaschen wenigstens 99 % ein genügend hohes Vakuum aufweisen.

Erfindungsgemäss wird diese Aufgabe gelöst durch die kennzeichnenden Merkmale des Anspruchs 1.

Anhand der beiliegenden schematischen Zeichnung wird die Erfindung beispielsweise erläutert. Es zeigen:

Fig. 1 das obere Ende einer Saugdrainageflasche mit aufgesetztem Stopfen in Ansicht,

Fig. 2 eine Draufsicht auf Fig. 1,

Fig. 3 eine Ansicht des Stopfens gemäss Fig. 1 von unten und

Fig. 4 einen Schnitt längs der Linie IV-IV in Fig. 3.

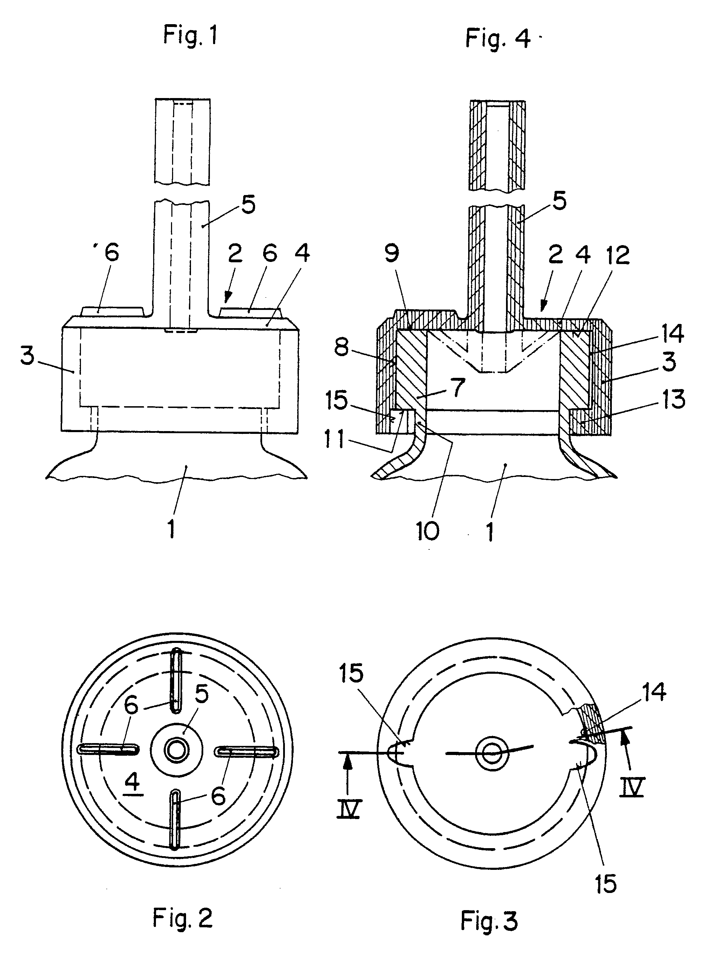

Auf eine Drainageflasche 1 ist ein vacuumdicht-schliessender Flaschenpfropfen 2 aufgesetzt. Der Flaschenpfropfen 2 besteht aus einem gummielastischen Material und weist einen Mantel 3 auf. Der Mantel 3 hat eine zylindrische Form und ist an einem Ende durch eine Wand 4 abgeschlossen. Die Wand 4 ist membranartig ausgebildet, so dass sie sich bei evakuierter Flasche gegen das Flascheninnere wölbt, wie dies in Fig. 2 strichpunktiert angedeutet ist. Zentral an die Wand 4 ist ein Schlauchstück 5 angeschlossen, das dem Anschluss einer zur Wunde führenden Drainageleitung dient. Diametral über die Wand 4 erstrecken sich Rippen 6, die die Sichtbarkeit einer Wölbung der Wand 4 wesentlich erhöht, so dass die Stärke des Vakuums in der Drainageflasche 1 visuell gut erkennbar ist.

Die Drainageflasche 1 weist eine Mündung 7 mit einer zylindrischen Aussenfläche 8 und einer ebenen Stirnfläche 9 auf. Die Mündung 7 bildet gegenüber dem Flaschenhals 10 eine hinterschnittene Schulter 11. Die Wand 4 des Stopfens 2 ist innenseitig als ebene Dichtungsfläche 12 gestaltet, der sie gegen die Stirnseite 9 der Flaschenmündung 7 dicht anliegt. Der Stopfen 2 hintergreift mit einer nach innen gerichteten Ringrippe 13 die Schulter 11 und ist dadurch unverlierbar auf der Flaschenmündung 7 gehalten. Parallel zur Mittelachse des Mantels 3 ist dieser innenseits mit einer Rippe 14 versehen, die sich von der Dichtungsfläche 12 bis zur Ringrippe 13 erstreckt. Die Rippe 14 hält die Mantelinnenseite auf Distanz zur zylindrischen Aussenfläche 8 der Flaschenmündung 7, so dass der Mantel 3 und die Flaschenmündung 7 nicht dicht aneinander liegen. Anstatt der Rippe 14 könnte auch auf der Mantelinnenseite oben und angrenzend an die Dichtfläche 12 lediglich eine Nocke vorhanden sein. Weiter sind in der Ringrippe 13 zwei Ausnehmungen 15 vorhanden, durch welche ein zwischen dem Mantel 3 und der Flaschenmündung 7 befindliches Gas nach aussen entweichen kann. Anstelle der Ausnehmungen 15 können auch ein oder mehrere radiale Bohrungen im Mantel 3 angebracht werden.

Wird die Drainageflasche 1 bei aufgesetztem Flaschenpfropfen 2 mit gesperrtem Schlauchstück 5 erhitzt, wölbt die sich im Flascheninneren befindliche Luft die Wand 4 nach aussen, wodurch die Luft zwischen die Flächen 9, 12 und von dort längs der Rippe 14 zwischen den Mantel 3 und die Flaschenmündung 7 treten und anschliessend durch die Ausnehmungen 14 entweichen kann.

Beim Abkühlen der Drainageflasche entsteht im Inneren ein Vacuum, wodurch die Fläche 12 gegen die Stirnfläche 9 der Flaschenmündung 7 gepresst wird, so dass der Flaschenpfropfen 2 die Drainageflasche vacuumdicht abschliesst.

Wird anstelle der Rippe 14 lediglich am oberen Ende der Mantelinnenseite, angrenzend an die Dichtfläche 12 nur ein einziger noppenartiger Vorsprung angebracht, so genügt dies, um während des Erhitzens der Flasche ein hinreichendes Abströmen der erhitzten Luft zu ermöglichen.

## Patentansprüche

1. Stopfen aus gummielastischem Material für eine Saugdrainageflasche mit einem im Querschnitt ringförmigen Mantel (3), der an einem Ende durch eine ein Schlauchstück ( 5) aufweisende Wand (4) abgeschlossen ist, die auf ihrer Innenseite eine Dichtfläche (12) aufweist, die mit der Flaschenmündung (7) zusammenwirkt und auf der anderen Seite mit einem nach innen gerichteten Ringwulst (13) versehen ist, wobei der Raum zwischen der Mantelinnenfläche und der Flaschenmündung (7) entlüftet ist, dadurch gekennzeichnet, dass die Dichtfläche (12) eben ist, dass bei auf die Saugdrainageflasche aufgesetztem Stopfen (2) die Dichtfläche (12) ausschliesslich mit der Stirnseite (9) der Flaschenmündung (7) zusammenwirkt, und dass der Innenquerschnitt des Mantels (3) derart gestaltet ist, dass die Mantelinnenfläche mindestens an einer Stelle des an die Dichtfläche (12) angrenzenden Bereichs nicht dicht gegen die Aussenseite der Flaschenmündung (7) anliegt.

2. Stopfen nach Anspruch 1, dadurch gekennzeichnet, dass auf der Mantelinnenseite, im Bereich der ebenen Dichtfläche (12) ein Vorsprung (14) vorhanden ist.

3. Stopfen nach Anspruch 2, dadurch gekennzeichnet, dass der Vorsprung (14) durch eine zur Mantellängsachse parallele Rippe gebildet ist.

4. Stopfen nach einem der Ansprüche 1 bis 3, dadurch gekennzeichnet, dass die Entlüftung der Mantelinnenseite durch eine Durchbrechung oder Ausnehmung (15) im Mantel oder im Wulst erfolgt.

5. Saugdrainageflasche mit einem Stopfen gemäss Anspruch 1, dadurch gekennzeichnet, dass die Flaschenmündung (7) eine ebene Stirnfläche (9) und eine zylindrische Aussenfläche (8) aufweist, und gegen den Flaschenhals (7) eine hinterschnittene Schulter (11) bildet.

**Claims**

1. Stopper made of rubber-elastic material for a suction drainage bottle, having a casing (3) which is annular in cross-section and is closed off at one end by means of a wall (4) with a tube piece (5), which wall (4) has on its inner side a sealing face (12), which cooperates with the bottle opening (7), and on the other side is provided with an inwardly directed annular bead (13), air being removed from the space between the inner face of the casing and the bottle opening (7), characterised in that the sealing face (12) is flat, in that, with the stopper (2) positioned on the suction drainage bottle, the sealing face (12) cooperates exclusively with the end face (9) of the bottle opening (7), and in that the internal cross-section of the casing (3) is designed in such a way that the inner face of the casing, at least at one site of the area adjoining the sealing face (12), does not bear tightly against the outside of the bottle opening (7).

2. Stopper according to Claim 1, characterised in that a projection (14) is present on the inner side of the casing, in the area of the flat sealing face (12).

3. Stopper according to Claim 2, characterised in that the projection (14) is formed by a rib parallel to the longitudinal axis of the casing.

4. Stopper according to one of Claims 1 to 3, characterised in that air is removed from the inside of the casing through a break or recess (15) in the casing or in the bead.

5. Suction drainage bottle with a stopper according to Claim 1, characterised in that the bottle opening (7) has a flat end face (9) and a cylindrical outer face (8), and forms towards the bottleneck (10) an undercut shoulder (11).

**Revendications**

1. Bouchon en une matière à l'élasticité du caoutchouc pour un flacon utilisé pour le drainage par aspiration, ayant une enveloppe (3) de section annulaire dont une extrémité est fermée par une paroi (4) munie d'un embout à tuyau (5), et ayant sur sa face intérieure une surface d'étanchéité (12) coopérant avec l'embouchure (7) de la bouteille et sur l'autre face un bourrelet annulaire (13) dirigé vers l'intérieur, le volume compris entre la surface-enveloppe et l'embouchure (7) de la bouteille étant vide d'air, bouchon caractérisé en ce que la surface d'étanchéité (12) est plane et lorsque le bouchon (2) est mis en place sur le flacon utilisé pour le drainage par aspiration, la surface d'étanchéité (12) coopère uniquement avec la surface frontale (9) de l'embouchure (7) de la bouteille et la section intérieure de l'enveloppe (3) a une forme telle que la surface-enveloppe intérieure, à au moins un endroit de la zone adjacente à la surface d'étanchéité (12) ne s'applique pas contre le côté extérieur de l'embouchure (7) du flacon.

2. Bouchon selon la revendication 1, caractérisé en ce que la face intérieure de l'enveloppe comporte une partie en saillie (14) au niveau de la surface d'étanchéité plane (12).

3. Bouchon selon la revendication 2, caractérisé en ce que la partie en saillie (14) est formée par une nervure parallèle à l'axe longitudinal de l'enveloppe.

4. Bouchon selon l'une des revendications 1 à 3, caractérisé en ce que le vide d'air du côté intérieur de l'enveloppe se fait par une interruption ou une cavité (15) dans l'enveloppe ou dans le bourrelet.

5. Flacon pour le drainage par aspiration comportant un bouchon selon la revendication 1, caractérisé en ce que l'embouchure (7) du flacon présente une surface frontale (9) plane et une surface extérieure cylindrique (8) ainsi qu'un épaulement (11) à contre-dépouille au niveau du col (7) du flacon.

Fig. 1

Fig. 4

Fig. 2

Fig. 3